(19) Europäisches Patentamt
European Patent Office
Office européen des brevets

(11) EP 1 289 977 B1

(12) EUROPEAN PATENT SPECIFICATION

(45) Date of publication and mention of the grant of the patent:
**23.03.2005 Bulletin 2005/12**

(21) Application number: **01931948.2**

(22) Date of filing: **23.05.2001**

(51) Int Cl.[7]: **C07D 307/00**, A61K 31/343, A61P 35/00

(86) International application number:
**PCT/GB2001/002341**

(87) International publication number:
**WO 2001/090096 (29.11.2001 Gazette 2001/48)**

# (54) TRITERPENOID DERIVATIVES AND THEIR USE AS ANTIPROLIFERATIVE AGENTS

TRITERPEN-DERIVATE UND IHRE VERWENDUNG ALS ANTIPROLIFERATIVE WIRKSTOFFE

DERIVES TRITERPENOIDES ET LEUR UTILISATION COMME AGENTS ANTIPROLIFERATIFS

(84) Designated Contracting States:
**AT BE CH CY DE DK ES FI FR GB GR IE IT LI LU MC NL PT SE TR**

(30) Priority: **23.05.2000 GB 0012532**
**25.05.2000 GB 0012825**

(43) Date of publication of application:
**12.03.2003 Bulletin 2003/11**

(73) Proprietors:
- **Univerzita palackeho V Olomouci 772 00 Olomouc (CZ)**
- **UNIVERZITA KARLOVA PRAHA 110 00 Praha 1 (CZ)**

(72) Inventors:
- **HAJDUCH, Marian 772 00 Olomouc (CZ)**
- **SAREK, Jan Ostrava-Poruba (CZ)**

(74) Representative: **Gabrielova, Marta Inventia s.r.o., Tr. Politickych veznu 7 110 00 Praha 1 (CZ)**

(56) References cited:
**WO-A-98/32443**

- **BARTON, D. H. R.; ET AL.: "Long-range Effects in Alicyclic Systems. Part II. ..." J. CHEM. SOC., 1957, pages 935-944, XP001018471**
- **DATABASE CROSSFIRE BEILSTEIN [Online] Beilstein Institut zur Förderung der Wissenschaften, Frankfurt am Main, DE; Database-Accession no. 6751453 (BRN), XP002177556 & RAO, K. ET AL.: J. INDIAN. CHEM. SOC., vol. 57, no. 8, 1980, pages 833-834,**
- **HUNECK, S.: "Die Darstellung von 19-beta,28-Epoxy-3-oxo-2-diazo-18-alpha-H-oleanan ..." CHEM. BER., vol. 98, 1965, pages 1837-1857, XP002177555 & DATABASE CROSSFIRE BEILSTEIN [Online] Beilstein Institut zur Förderung der Wissenschaften, Frankfurt am Main, DE; Database-Accession no. 1335132 (BRN),**
- **DATABASE CROSSFIRE BEILSTEIN [Online] Beilstein Institut zur Förderung der Wissenschaften, Frankfurt am main, DE; database-Accession no. 1668344 (BRN), XP002177557 & KLINOT, J. ET AL.: "Prearation of 2-Methyl-3-oxo Triterpenoids ..." COLLECT. CZECH. CHEM. COMMUN., vol. 44, 1979, pages 211-225, XP001021678**
- **HUNECK, S.: "Photochemische Umsetzungen. ..." TETRAHEDRON LETT., vol. 6, 1963, pages 375-379, XP001018476 & DATABASE CROSSFIRE BEILSTEIN [Online] Beilstein Institut zur Förderungen der Wissenschaften, Frankfurt am Main, DE; Database-Accession no. 1668960 (BRN), & DATABASE CROSSFIRE BEILSTEIN [Online] Beilstein Institut zur Förderung der Wissenschaften, Frankfurt am Main, DE; Database-Accession no. 1670459 (BRN),**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 289 977 B1

- DATABASE CROSSFIRE BEILSTEIN [Online] Beilstein Institut zur Förderung der Wissenschaften, Frankfurt am Main, DE; Database-Accession no. 3175536 (BRN), XP002177558 & SCHULZE, H.; PIEROH, K.: "Zur Kenntnis des Betulins." CHEM. BER., vol. 55, 1922, pages 2332-2346,
- DISCHENDORFER, O.; JUVAN, H.: "Untersuchungen auf dem Gebiete der Phytochemie" MONATSH. CHEM., vol. 56, 1930, pages 272-281, XP001018470 & DATABASE CROSSFIRE BEILSTEIN [Online] Beilstein Institut zur Förderung der Wissenschaften, Frankfurt am Main, DE; Database-Accession no. 60664 (BRN),
- PLATANOV V.G. ET AL KHIM.-FARM. ZH. vol. 29, no. 2, 1995, pages 42 - 46, XP001021510

## Description

The present invention relates to the therapeutic use and the biological activity of triterpenoid derivatives. The invention further relates to novel triterpenoid derivatives.

To date, the prior art has primarily focussed on compounds that are capable of regulating the cell cycle by virtue of inhibiting cyclin dependent kinases (CDKs). Examples of such compounds include butyrolactone I, flavopiridol, bohemin, olomoucine, roscovitine, purvanalol and indarubicine.

There is considerable support in the literature for the hypothesis that CDKs and their regulatory proteins play a significant role in the development of human tumours. Thus, in many tumours a temporal abnormal expression or activity of CDKs has been observed, together with a major deregulation of protein inhibitors (mutations, deletions). This results in the activation of CDKs and consequently in defective regulation of the Gl/S transition. Unlike normal cells, tumour cells do not arrest in G1, and since they become independent of growth factors, they pass the G1 restriction point and enter the S phase very rapidly.

In contrast to the prior art, the present invention relates to compounds which are anti-proliferative, but which are believed to operate via a mechanism other than CDK inhibition.

The $G_1$/S transition of the mammalian cell cycle is tightly regulated by the retinoblastoma protein (pRb). Retinoblastoma gene mutations or deletions predispose individuals to familiar retinoblastoma and other types of cancers. The pRb protein is a docking protein, which in hypophosphorylated form has the capacity to bind and thus to inactivate S-phase transcription factors such as DP-1 and E2F. However, following phosphorylation by $G_1$/S cyclin-dependent kinases (CDKs) (CDK4/cyclin D1-D3, CDK6/cyclin D1-D3, CDK2/cyclin A), hyperphosphorylated pRb releases the transcription factors and S phase is initiated. Within the S phase, the pRb protein phosphorylation is maintained by the activity of CDK2/cyclin E complexes. Thus, hyperphosphorylation of the pRb protein plays a key role in the molecular pathology of cancer cells with altered CDK activity.

The present invention relates to the use of triterpenoid compounds derived from the natural products betulin and betulinic acid (BA) as shown in formula (A). The compounds of the present invention are referred to hereinafter as betulinines.

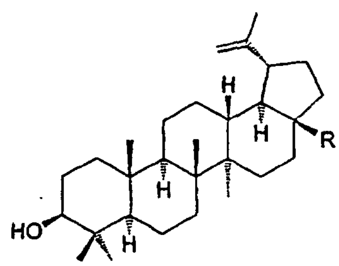

R = $CH_2OH$: Betulin
R = COOH: Betulinic acid

(A)

With regard to their biological and therapeutic activity, the compounds disclosed herein are believed to be of specific benefit in the treatment of proliferative diseases such as cancers and leukaemias.

Several of the compounds suitable for use in the present invention are already known in the art, for example those disclosed in Collect. Czech. Chem. Commun. 52, 1052 (1987), Sejbal J. *et al.*; Hunech, S. Chem. Ber. 98, 1837 (1965); Klinot, J. *et al.* Collect. Czech. Chem. Commun. 44, 211 (1979); and Huneck, S. Tetrahectron Lett. 6, 375 (1963). However, these disclosures do not include any indication as to possible biological activity of such compounds. WO 98/32443 relates to pharmaceutical formulations of esters of pentacyclic triterpenes such as allobetuline esters of antitumour. antiviral, and antiparasitic activity.

A first aspect of the present invention relates to a compound of formula I, or a pharmaceutically acceptable salt thereof, for use in therapy

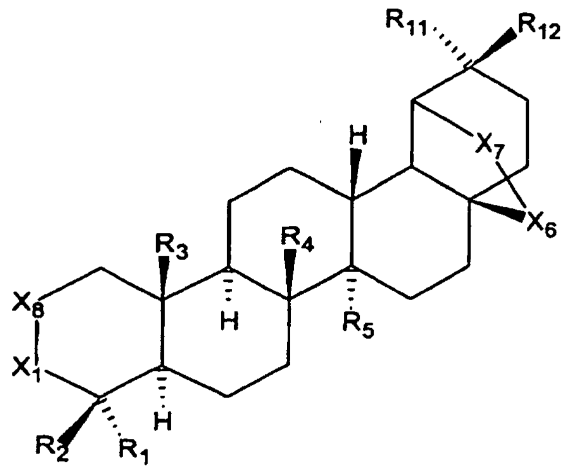

I

wherein:

$X^1$ is C=O;
$X^6$ is C=O, $CH_2$;
$X^7$ is O;
$X^8$ is C=O, C=NOR$^{1b}$, CHCN, CHOR$^{1b}$, CHOCOR$^{1b}$, CHOC(O)OR$^{1b}$, CHOC(O)OR$^{13}$, $CHOSO_2CH_3$, $CHOSO_2C_6H_4CH_3$, CH-Hal, C-(Hal)$_2$, $CHN_3$, $CH_2$, C=C(R$^{1b}$)$_2$, C=CR$^{1b}$OR$^{1b}$;
$R^{2-5}$ and $R^{11}$, $R^{12}$ are Me,
and wherein $R^1$ is H or lower alkyl, and Hal is Br, Cl, I, F;
$R^{1a-1b}$ are the same or different groups of $R^1$
$R^{13}$ is an OH-substituted alkyl group, an ether group or a cyclic ether.

In a preferred aspect, the invention relates to a compound of formula I, or a pharmaceutically acceptable salt thereof, for treating a patient suffering from leukaemia, cancer or other proliferative disorder.

A second aspect of the present invention relates to novel betulinines of structural formula Ia, or pharmaceutically acceptable salts thereof,

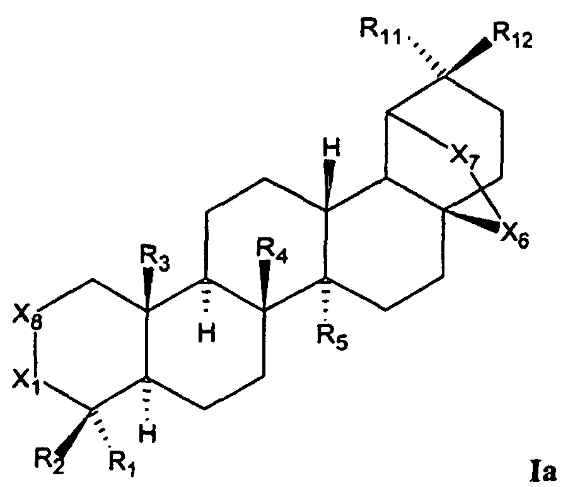

Ia

wherein:

$X^1$ is C=O;
$X^6$ is C=O, $CH_2$;
$X^7$ is O;
$X^8$ is C=O, $C=NOR^{1b}$, CHCN, $CHOR^{1b}$, $CHOCOR^{1b}$, $CHOC(O)OR^{1b}$, $CHOC(O)OR^{13}$, $CHOSO_2CH_3$, $CHOSO_2C_6H_4CH_3$, CH-Hal, C-$(Hal)_2$, $CHN_3$, $C=C(R^{1b})_2$, $C=CR^{1b}OR^{1b}$;
$R^{2-5}$ and $R^{11}$, $R^{12}$ are Me,
and wherein $R^1$ is H or lower alkyl, and Hal is Br, Cl, I, F;
$R^{1a-1b}$ are the same or different groups of $R^1$;
$R^{13}$ is an OH-substituted alkyl group, an ether group or a cyclic ether;
with the proviso that when $X^6$ is $CH_2$, $X^7$ is O, $R^{1-5}$, $R^{11}$ and $R^{12}$ are Me;
$X^8$ is other than C=O, C=NOH, C=CHOH, CHBr, CHOH, CHOAc, CHCl, CHOMe or CHOEt.

As used herein, the term lower alkyl means a linear or branched chain alkyl group containing from 1 to 6 carbon atoms, including methyl, ethyl, propyl, isopropyl, butyl, isobutyl, tertiary butyl.

**Description of the Preferred Embodiments**

Within the options provided for the groups $X^1$, $X^{6-8}$, $R^{1-5}$ and $R^{11-12}$ of formula I, the following options are preferred:

$$X^8 \text{ is C=O, CH-Hal, } CHOR^{1a}, CHOCOR^{1a}, C=C(R^{1b})_2, C=CR^{1b}OR^{1b}, C\text{-}Hal_2,$$

$$CHN_3 \text{ or } CHCOOR^{13}.$$

Preferably, $R^{13}$ is a diol, triol or polyol.
Even more preferably, $R^{13}$ is glycerol, 2,2-dimethyl-1,3-dioxolan-4-yl or pentaerythrityl.
In an even more preferred embodiment,
$X^8$ is selected from C=O, CHBr, CHOH, CHOAc, $C=CH_2$, C=CHOH, C-$Br_2$, $CHN_3$, $CHOC(O)OCH_2CHOHCH_2OH$ or $CHOC(O)OCH_2$-2,2-dimethyl-1,3-dioxolan-4-yl.
In a particularly preferred embodiment of the first aspect of the invention, the compounds of use are selected from those shown in Table 1 below:

Table 1

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.3 | C=O | $CH_2$ | O | CHBr | Me |
| III.4 | C=O | $CH_2$ | O | CHOH | Me |
| III.5 | C=O | $CH_2$ | O | CHOAc | Me |
| III.10 | C=O | $CH_2$ | O | $CHN_3$ | Me |
| III.13 | C=O | $CH_2$ | O | $CBr_2$ | Me |
| III.14 | C=O | $CH_2$ | O | $CF_2$ | Me |
| III.16 | C=O | $CH_2$ | O | CHF | Me |
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C=CH_2$ | Me |
| III.32 | C=O | $CH_2$ | O | C=CHOH | Me |

where

A is $CHOC(O)OCH_2CHOHCH_2OH$ and
B is $CHOC(O)OCH_2$-2,2-dimethyl-1,3-dioxolan-4-yl.

In a particularly preferred embodiment of the invention, said compound is of the formula

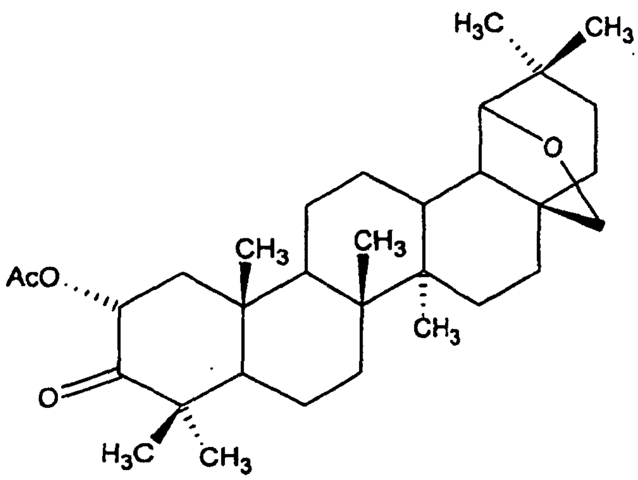

In respect of the second aspect of the invention, the preferred embodiments regarding the compounds are identical to those given above for the first aspect with application of the proviso of formula Ia.

The most preferred compounds of the second aspect are those in Table 1a below:

Table 1a

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.10 | C=O | $CH_2$ | O | $CHN_3$ | Me |
| III.13 | C=O | $CH_2$ | O | $CBr_2$ | Me |
| III.14 | C=O | $CH_2$ | O | $CF_2$ | Me |
| III.16 | C=O | $CH_2$ | O | CHF | Me |
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C=CH_2$ | Me |

where

A is $CHOC(O)OCH_2CHOHCH_2OH$ and
B is $CHOC(O)OCH_2$-2,2-dimethyl-1,3-dioxolan-4-yl.

In respect of the invention as a whole, it is preferable that the proliferative disorder is cancer or leukaemia. In one embodiment, the cancer or leukaemia is p53, hormone and multidrug resistance independent. In another embodiment, the cancer or leukaemia is independent of Rb status.

More specifically, the present invention relates to a method of treating patients suffering from cancer by administering therapeutically effective amounts of a compound of formula I or pharmaceutically acceptable salts or esters thereof.

Without wishing to be bound by theory, preliminary studies suggest that rather than influencing the activity of cyclin dependent kinases; the compounds of the present invention appear to operate via an alternative mechanism. In particular, it is believed that the betulinines of the present invention may inhibit cell proliferation and induce cancer cell death in a manner which involves mainly post-translational modifications, namely the phosphorylation, of a key regulatory protein involved in cellular proliferation. More specifically, it is believed that the betulinines of the invention effect a change in the phosphorylation state of the Rb protein. Such a mechanism may be advantageous as it is thought that the compounds of the present invention may be capable of inhibiting cell proliferation in proliferating tumour tissue, but not in healthy tissue.

Thus, in a further embodiment the present invention relates to a method of treating a cancerous or leukaemic proliferative disease through effecting a change in the pRb protein phosphorylation state by the administration of a therapeutically effective amount of a compound of formula I, or pharmaceutically acceptable salts or esters thereof.

The compounds of the present invention are also capable of inducing apoptosis (programmed cell death) in proliferative cells. Thus, in an additional embodiment, the present invention relates to a method of inducing cell death in proliferative cells comprising administering a therapeutically effective amount of a compound of formula I or pharmaceutically acceptable salts or esters thereof.

A further aspect of the present invention relates to use of betulinines of formula I as research chemicals and as compounds for clinical and/or laboratory diagnostics.

More particularly, the invention relates to the use of betulinines as research chemicals for studying the phosphorylation/de-phosphorylation processes of cellular substrates, cellular proliferation, purification of target molecules, and/or cell cycle studies.

The present invention therefore further relates to the use of a compound of formula I in the manufacture of a medicament for use in the treatment of a proliferative disease.

As used herein the phrase "manufacture of a medicament" includes the use of a compound of formula I directly as the medicament in addition to its use in a screening programme for further anti-proliferative agents or in any stage of the manufacture of such a medicament.

Such a screening programme may for example include an assay for determining the phosphorylation state of cellular substrates and determining whether a candidate substance is capable of mimicking the activity of a betulinine of formula I.

Thus, in a further embodiment, the invention relates to the use of a compound of formula I or a pharmaceutically acceptable salt, crystal form, complex, hydrate, or hydrolysable ester thereof, in an assay for determining the phosphorylation state of cellular substrates, and optionally in the identification of candidate compounds that act in a similar manner.

Preferably, the cellular substrate, the phosphorylation state of which is being assayed is Rb protein.

Such assays may be carried out by incubating a betulinin either alone or together with a candidate substance with a relevant cell line and assessing the phosphorylation profile the Rb protein over a period of time. If a candidate substance is present it's effect on the activity of the control betulinin will be evident by running the corresponding controls (betulinin alone and candidate alone). Further information on such assays including appropriate cell lines, reagents and Rb antibodies is given below.

Rb phosphorylation assay;

Since Rb protein contains multiple phosphorylation sites for CDKs, its phosphorylated form has molecular weight about 110 kDa, while the molecular weight of hypophosphorylated protein is only 105 kDa. This small difference in molecular weight is enough to separate both forms by conventional SDS-PAGE electrophoresis.

CEM cells may are cultured in Dulbeco's modified essential medium with 4.5 g dextrose/l, 10% of foetal calf serum, 2 mM glutamine, 100 U/ml penicillin and 100 μg/ml streptomycin with/without below indicated concentrations of betulinin. At selected time points, cells are harvested, washed in ice cold Hank's balanced salt solution and solubilized on ice using the SDS-PAGE sample buffer containing protease and phosphatase inhibitors (10 μg/ml of leupeptin, 10 μg/ml of aprotinin, 10 μg/ml of soybean trypsin inhibitor, 100 μmol of benzamide, 1 mM of sodium vanadate, 1 mM of NaF, 1 mM of phenylphosphate) and boiled immediately.

Total cellular proteins (100 μg/well) are separated on SDS-PAGE electrophoresis, blotted on polyvinyldifluoride membranes and total Rb protein, including proteolytic fragment(s) detected using a pRb monoclonal antibody (Oncogene, Germany, Rb(Ab-5), Cat# OP66 Rev 02-Sept-96 EB, Clone LM95.1) and visualized by chemiluminiscence (ECL-Western Blotting System, Amersham). Details of the Western blot technique are described in Ausubel, F.M., Brent, R., Kingston, R.E., Moore, D.D., Seidman, J.G., Smith, J.A., Struhl, K (Eds): Short Protocols in Molecular Biology, 2nd edition, John Wiley & Sons, New York, Chichester, Brisbane, Toronto, Singapore, 1992, page 10-33 - 10-35.

The compounds of the present invention can be present as salts or esters, in particular pharmaceutically acceptable salts or esters.

Pharmaceutically acceptable salts of the product of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as ($C_1$-$C_4$)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of I to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as ($C_1$-$C_4$)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

In all aspects of the present invention previously discussed, the invention includes, where appropriate all enantiomers and tautomers of compounds of formula I or Ia. The man skilled in the art will recognise compounds that possess optical properties (one or more chiral carbon atoms) or tautomeric characteristics. The corresponding enantiomers and/or tautomers may be isolated/prepared by methods known in the art.

The invention furthermore relates to the compounds of, or of use, in the present invention in their various crystalline forms, polymorphic forms and (an)hydrous forms. It is well established within the pharmaceutical industry that chemical compounds may be isolated in any of such forms by slightly varying the method of purification and or isolation from the solvents used in the synthetic preparation of such compounds.

The invention further includes the compounds of, or of use, in the present invention in prodrug form. Such prodrugs are generally compounds of formula I or Ia wherein one or more appropriate groups have been modified such that the modification is reversed upon administration to a human or mammalian subject. Such reversion is usually performed by an enzyme naturally present in such subject, though it is possible for a second agent to be administered together with such a prodrug in order to perform the reversion in vivo. Examples of such modifications include esters (for example, any of those described above), wherein the reversion may be carried out be an esterase etc. Other such systems will be well known to those skilled in the art.

The present invention also encompasses pharmaceutical compositions comprising the compounds of the invention. In this regard, and in particular for human therapy, even though the compounds of the present invention (including their pharmaceutically acceptable salts, esters and pharmaceutically acceptable solvates) can be administered alone, they will generally be administered in admixture with a pharmaceutical carrier, excipient or diluent selected with regard to the intended route of administration and standard pharmaceutical practice.

Thus, the present invention also relates to pharmaceutical compositions comprising betulinines or pharmaceutically acceptable salts or esters thereof, together with at least one pharmaceutically acceptable excipient, diluent or carrier.

By way of example, in the pharmaceutical compositions of the present invention, the compounds of the invention may be admixed with any suitable binder(s), lubricant(s), suspending agent(s), coating agent(s), and/or solubilising agent(s). Examples of such suitable excipients for the various different forms of pharmaceutical compositions described herein may be found in the "Handbook of Pharmaceutical Excipients, 2nd Edition, (1994), Edited by A Wade and PJ Weller.

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules. Preferably, these compositions contain from 1 to 250 mg and more preferably from 10-100 mg, of active ingredient per dose.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The

active ingredient can also be incorporated, at a concentration of between 1 and 10% by weight, into an ointment consisting of a white wax or white soft paraffin base together with such stabilisers and preservatives as may be required.

Injectable forms may contain between 10 - 1000 mg, preferably between 10 - 250 mg, of active ingredient per dose.

Compositions may be formulated in unit dosage form, i.e., in the form of discrete portions containing a unit dose, or a multiple or sub-unit of a unit dose.

A person of ordinary skill in the art can easily determine an appropriate dose of one of the instant compositions to administer to a subject without undue experimentation. Typically, a physician will determine the actual dosage which will be most suitable for an individual patient and it will vary with the age, weight and response of the particular patient. The dosages disclosed herein are exemplary of the average case. There can of course be individual instances where higher or lower dosage ranges are merited, and such are within the scope of this invention.

In an exemplary embodiment, one or more doses of 10 to 150 mg/day will be administered to the patient for the treatment of malignancy.

The invention further relates to methods of chemical synthesis of the above described compounds.

In one embodiment, the invention relates to a process for preparing a compound of formula I, as defined above, wherein $X^6$ is C=O,

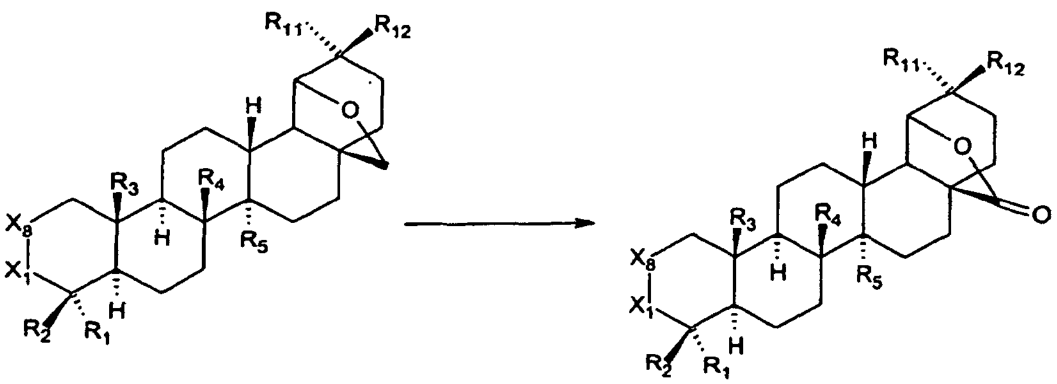

Ib Ic

comprising oxidising a compound of formula Ib to a compound of formula Ic.

In a preferred embodiment the compound of formula Ib is oxidised to a compound of formula Ic by treating with chromium trioxide.

The preparation of the compounds of the present invention will be discussed in greater detail below, with specific reference to the preferred embodiments. The man skilled in the relevant art would be able to prepare other compounds of the invention by selection of the appropriate reagents.

The following scheme illustrates the synthesis of compounds of formula I where $R^1$-$R^5$, $R^{11}$, $R^{12}$ are methyls, $X^7$ is oxygen, $X^6$ is C=O, $X^1$ is C=O and $X^8$ is CHOAc.

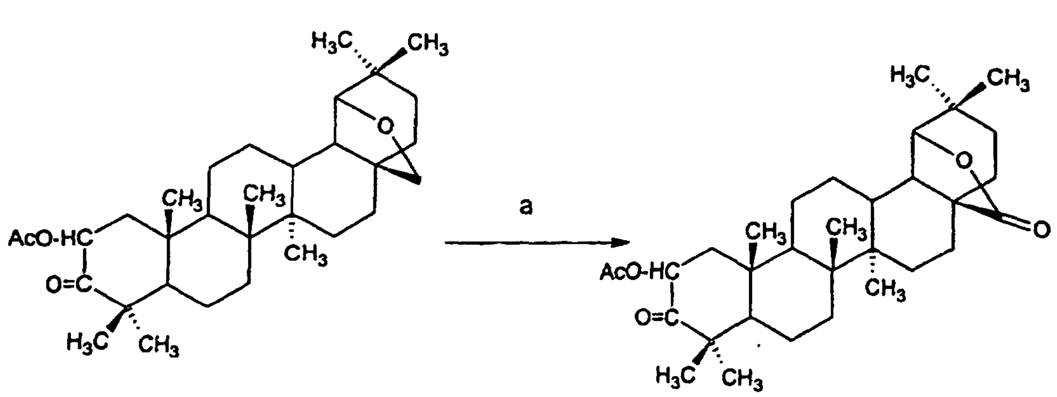


*Conditions:* a, oxidation (*e.g.* with chromium trioxide)

The scheme below illustrates the synthesis of compound III.10 of formula I where $R^1$-$R^5$, $R^{11}$, $R^{12}$ are methyls, $X^7$ is oxygen, $X^6$ is $CH_2$.

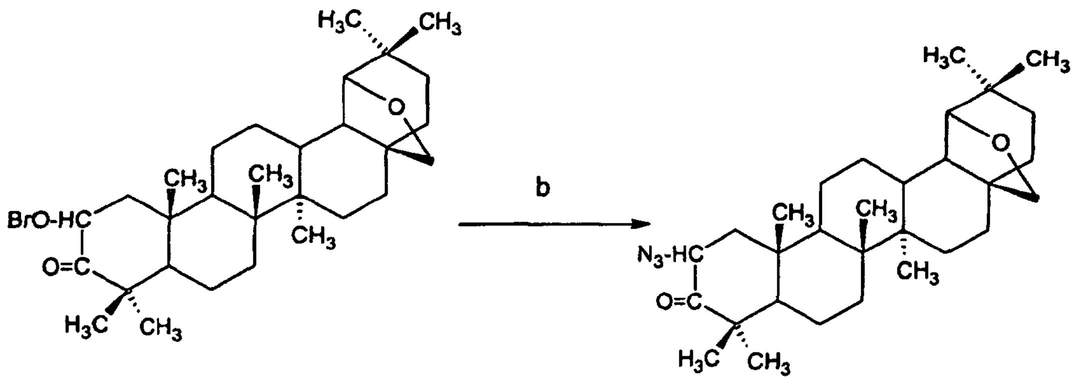


Id Ie

*Conditions:* b, nucleophilic substitution with sodium azide *(e.g.* in N-methylpyrrolid-2-one).

The scheme below illustrates the synthesis of compounds III.21 and III.22 of formula I where $R^{1-5}$, $R^{11,12}$ are methyls, $X^7$ is oxygen, $X^6$ is $CH_2$.

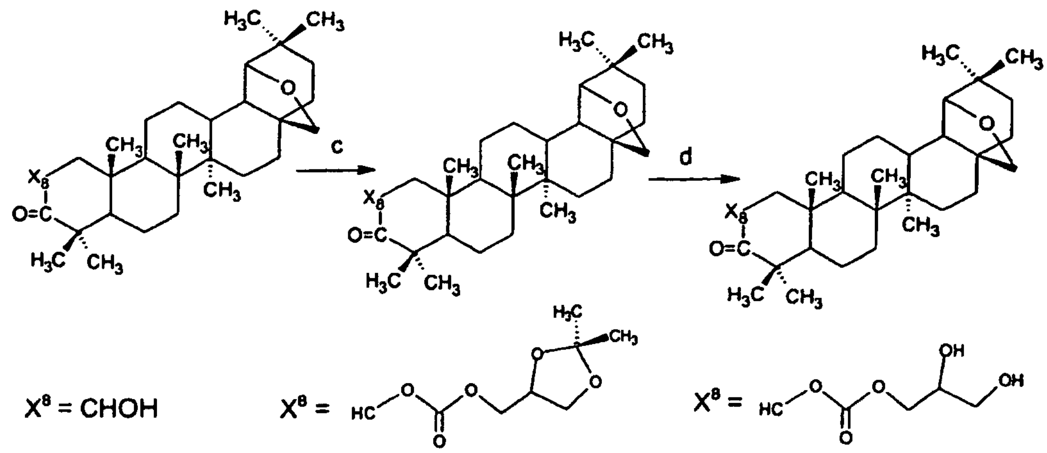


If Ig Ih

*Conditions:* c, esterification with chloroformate of solketal in presence of base (*e.g.* pyridine); d, deprotection of acetonide (*e.g.* with hydrochloric acid).

The scheme below illustrates the synthesis of compound III.14 of formula I where $R^1$-$R^5$, $R^{11}$, $R^{12}$ are methyls, $X^7$ is oxygen, $X^6$ is $CH_2$.

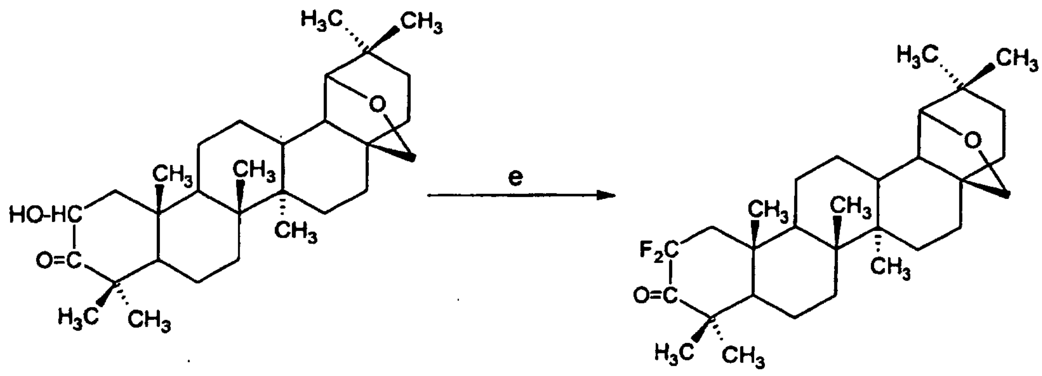


Ii Ij

*Conditions:* e, reaction with diethylaminosulphur trifluoride.

This invention is further illustrated by the following examples, which should not be construed as further limiting.

## EXAMPLES

### General

The chemical shift values (δ-scale, ppm) and coupling constants (J, Hz) in the $^{1}H$ and $^{13}C$ NMR spectra were obtained using a Varian UNITY-INOVA 400 FT spectrometer ($^{1}H$ at 400 MHz and $^{13}C$ at 100.6 MHz) in deuterochloroform with tetramethylsilane (for $^{1}H$ NMR data δ = 0 ppm) as an internal standard. For the $^{13}C$ NMR data δ($CDCl_3$) = 77.00 ppm. The value for a multiple!, either defined (doublet (d), triplet (t), quartet (q), septet (sept.)) or not (m), at the approximate mid point is given unless a range is quoted (s = singlet, b = broad).

Electron impact mass spectra (EIMS) were measured on an INCOS 50 instrument. Ionising electron energy 75 eV, ion source temperature 150°C. EIMS was used to determine molecular weights, $M^+$ corresponding to the molecular ion.

Ether is diethylether. THF and dioxane were dried over sodium. Acetic acid was purified before use by chromium trioxide treatment and distillation. Reactions were run at room temperature unless otherwise stated. The reaction progress was monitored by thin layer chromatography (TLC) on silicagel 60 G (Merck, detection by spraying with 10 % sulphuric acid and heating). The work-up procedure involves dilution with specified solvent (otherwise the organic reaction solvent), extraction with water and then brine or sodium hydrogencarbonate, drying over anhydrous magnesium sulphate, and evaporation under vacuum to give a residue.

### EXAMPLE 1

#### 2α-acetoxy-3-oxo-18α-oleanan-28,19β-olide

Chromium trioxide (2 g; 20 mmol) was added to a solution of 3-oxo-19β,28β-epoxy-18α-oleanan-2-yl acetate [Sejbal J., Klinot J., Vystrcil A.: Collect. Czech. Chem. Commun. 52, 1052 (1987)] (1 g; 2 mmol) in acetic acid (15 ml). The mixture was stirred for 3 hours at a higher temperature. The resulting precipitate was filtered off and washed with acetic acid and methanol. The yield of white crystalline solid was 0.3 g (30%), m.p.315 - 317°C, $[\alpha]_D$ +47° (c 0.29; $CHCl_3$).

The $^{13}C$ NMR spectrum of the title compound is as follows: 209.2, 179.7, 170.1, 85.9, 71.8, 57.4, 50.9, 48.7, 46.7, 46.2, 46.1, 40.7, 40. 1, 38.2, 35.9, 33.6, 33.4, 32.3, 31.9, 28.7, 27.8, 26.3, 25.5, 24.7, 23.9, 21.2, 21.0, 20.8, 18.9, 17.0, 15.7, 13.6.

### EXAMPLE 2

#### 2-azido-3-oxo-19β,28β-epoxy-18α-oleanan

Sodium azide (0.75 g; 11.6 mmol) was added to a solutiom of 2-brom-3-oxo-19β,28β-epoxy-18α-oleanan [Klinot J., Vystrcil A.: Collect. Czech. Chem. Commun. 31, 1079 (1966)] (1 g; 1.9 mmol) in N-methylpyrrolidone (20 ml) and acetic acid (1 ml). The mixture was stirred for 19 hours at room temperature. The product was worked up by general

manner and crystallized from ethyl acetate. The yield of pale yellow crystalline solid was 0.5 g (54%), m.p. 94 -97°C, $[\alpha]_D$ +91° (c 0.50; $CHCl_3$).

The $^{13}C$ NMR spectrum of the title compound is as follows: 213.4, 210.6, 87.9, 87.8, 71.2, 71.2, 60.9, 59.9, 57.1, 52.2, 50.6, 50.4, 48.7, 47.9, 46.8, 46.7, 46.7, 46.5, 41.4, 41.4, 40.9, 40.8, 40.8, 40.4, 37.9, 37.1, 36.7, 36.7, 36.3, 36.3, 34.4, 34.1, 33.5, 32.7, 32.7, 32.4, 29.3, 28.8, 28.8, 26.4, 26.4, 26.4, 26.2, 26.2, 26.2, 24.9, 24.5, 24.5, 22.1, 21.4, 21.3, 19.9, 19.5, 19.0, 18.7, 16.7, 15.8, 15.0, 13.4, 13.4.

EXAMPLE 3

19β,28β-epoxy-3-oxo-18α-oleanan-2α-yl [(2,2-dimethyl-1,3-dioxolan-4-yl)methyl] carbonate

A solution of 19β,28β-epoxy-2α-hydroxy-18α-oleanan-3-one (0.2 g; 0.4 mmol) in a mixture of dichloromethane (3.2 ml), pyridine (0.16 ml) and chloroformate of solketal (0.32 ml; 1.6 mmol) was vigorously stirred for 3 hours at room temperature. After the reaction was complete, the mixture was slowly poured into 50 ml of 5 % hydrochloric acid with ice, twice extracted with chloroform. The combined organic layers were worked up in the usual manner and chromatographed on silica gel (20 % ethyl acetate in hexane). The yield of snow-white lyophilized (from 2-methyl-2-propanol) powder was 0.19 g (70%), m.p. 192 -194°C, $[\alpha]_D$ +47° (c 0.54; $CHCl_3$).

The $^{13}C$ NMR spectrum of the title compound is as follows: 208.7, 155.4, 154.4, 109.9, 109.8, 87.9, 75.3, 73.2, 73.1, 71.2, 67.8, 66.5, 66.4, 57.3, 50.8, 48.6, 46.8, 46.0, 41.4, 40.9, 40.8, 38.2, 36.3, 36.7, 34.1, 33.5, 32.7, 28.8, 26.8, 26.7, 26.4, 26.2, 26.2, 25.4, 25.3, 24.6, 24.5, 21.3, 21.0, 18.9, 17.0, 15.8, 13.4.

EXAMPLE 4

19β,28β-epoxy-3-oxo-18α-oleanan-2α-yl [(2,3-dihydroxypropyl] carbonate

Dilute aqueous HCl (10 %; 1.5 ml) was added to a solution of 19β,28β-epoxy-18α-oleanan-3-oxo-2α-yl [(2,2-dimethyl-1,3-dioxolan-4-yl)methyl] carbonate (0.1 g; 0.2 mmol) in THF (3 ml) and the resulting clear reaction mixture was stirred for ten hours at 30°C. After the reaction was complete, the mixture was diluted with cold water (50 ml) and twice extracted with ethyl acetate. The combined organic layers were worked up in the usual manner and chromatographed on silica gel (75 % ethyl acetate in hexane). The yield of white lyophilized (from 2-methyl-2-propanol) solid was 0.069 g (74%), m.p. 174 -176°C, $[\alpha]_D$ +43° (c 0.53; $CHCl_3$).

The $^{13}C$ NMR spectrum of the title compound is as follows: 209.3, 154.8, 87.9, 75.5, 71.2, 69.9, 69.8, 68.9, 68.7, 63.0, 57.3, 50.8, 48.7, 46.7, 46.0, 41.4, 40.9, 40.8, 38.3, 36.7, 36.3, 34.1, 33.5, 32.7, 28.8, 26.4, 26.2, 26.2, 24.5, 24.5,21.3, 21.0, 18.9, 17.0, 15.8, 13.4.

EXAMPLE 5

19β,28β-epoxy-2,2-difluoro-18α-oleanan-3-one

Diethylaminosulphur trifluoride (DAST, 0.2 ml; 1.3 mmol) was added dropwise to a solution of 19β,28β-epoxy-2α-hydroxy-18α-oleanan-3-one (0.2 g; 0.4 mmol) in dry chloroform (2 ml) and the reaction mixture was stirred overnight at room temperature. After the reaction was complete, the mixture was slowly poured into cold water (50 ml) and twice extracted with chloroform. The combined organic fractions were worked up in the usual manner and chromatographed on silica gel (10 % ethyl acetate in hexane). The residue was crystallized from isopropyl alcohol to give 0.07 g (36%) of the title compound as pearl petals, m.p. 243 -245°C, $[\alpha]_D$ +123° (c 0.52; $CHCl_3$).

The $^{13}C$ NMR spectrum of the title compound is as follows: 204.6 dd ($J^1$ = 25.2, $J^2$ = 22.5), 115.8 dd ($J^1$ = 257.9, $J^2$ = 244.6), 87.9 s, 71.2 s, 51.8 dd ($J^1$ = 22.5, $J^2$ = 20.2), 50.9 s, 50.1 s, 46.7 s, 45.9 s, 41.4 s, 40.8 s, 40.5 s, 37.1 dds ($J^1$ = 5.3, $J^2$ = 2.3), 36.7 s, 36.3 s, 34.3 s, 32.7 s, 32.3 s, 28.8 s, 28.1 d($J^1$ = 3.8), 26.4 s, 26.3 s, 26.1 s, 24.5 s, 21.9 s, 21.0 d ($J^1$ =1.1), 19.6 s, 18.5 s, 15.1 s, 13.4 s.

EXAMPLE 6

BIOLOGICAL ACTIVITY OF BETULININES

In vitro cytotoxic activity of betulinines on tumor cell lines

One of the parameters used as the basis for colorimetric assays is the metabolic activity of viable cells. For example, a microtiter assay which uses the tetrazolium salt MTT is now widely used to quantitate cell proliferation and

cytotoxicity [Hajduch M, Mihal V, Minarik J, Fáber E, Šafárová M, Weigl E, Antálek P.: Cytotechnology, 1996,19,243-245]. For instance, this assay is used in drug screening programs and in chemosensitivity testing. Because tetrazolium salts are cleaved only by metabolically active cells, these assays exclusively detect viable cells. In the case of the MTT assay, yellow soluble tetrazolium salt is reduced to a coloured water-insoluble formazan salt. After it is solubilized, the formazan formed can easily and rapidly be quantified in a conventional ELISA plate reader at 570 nm (maximum absorbancy). The quantity of reduced formazan corresponds to the number of vital cells in the culture.

Human T-lymphoblastic leukaemia cell line CEM was used for routine screening of these compounds. To prove a common mechanism of action, selected compounds which showed activity in a screening assay were tested in a panel of cell lines (Table 2). These lines were from different species and of different histogenetic origin and they possess various alterations in cell cycle regulatory proteins and hormone dependence status (Table 2). The cells were maintained in Nunc/Coming 80 $cm^2$ plastic tissue culture flasks and cultured in cell culture medium (DMEM with 5 g/l glucose, 2mM glutamine, 100 U/ml penicillin, 100 μg/ml streptomycin, 10% foetal calf serum and sodium bicarbonate). Individual compounds were dissolved in 10% dimethylsulfoxide/saline, pH 8.0.

The cell suspensions that were prepared and diluted according to the particular cell type and the expected target cell density (2.500-30.000 cells per well based on cell growth characteristics) were added by pipette (80 μl) into 96/well microtiter plates.

Inoculates were allowed a pre-incubation period of 24 hours at 37°C and 5% $CO_2$ for stabilisation. Four-fold dilutions of the intended test concentration were added at time zero in 20 μl aliquots to the microtiter plate wells. Usually, test compounds were evaluated at six 4-fold dilutions. In routine testing, the highest well concentration was 250 μM, but it may differ, depending on the agent. All drug concentrations were examined in duplicate. Incubations of cells with the test compounds lasted for 72 hours at 37°C, in 5% $CO_2$ atmosphere and 100% humidity. At the end of the incubation period, the cells were assayed by using the MTT assay. Ten microliters of the MTT stock solution were pipetted into each well and incubated further for 1-4 hours. After this incubation period, formazan was solubilized by the addition of 100μl/well of 10% SDS in water (pH=5.5) followed by further incubation at 37°C overnight. The optical density (OD) was measured at 540nm with the Labsystem iEMS Reader MF (UK). The tumour cell survival (TCS) was calculated using the following equitation: TCS= ($OD_{drug\ exposed\ well}$ /mean $OD_{control\ wells}$) x 100%. The $TCS_{50}$ value, the drug concentration lethal to 50% of the tumour cells, was calculated from the obtained dose response curves.

To evaluate the anti-cancer activity of betulinines, their cytotoxic activity against CEM cell line was examined using the screening assay. Potent compounds were further tested against a panel of cell lines of different histogenetic and species origin (Table 2).

Table 2

| Cytotoxic activity of selected betulinines against a panel of different (non)malignant cell lines. | | | | | |
|---|---|---|---|---|---|
| Cell Line | Description | Compound ($TCS_{50}$[mM]) | | | |
| | | Betulinic Acid | III.3 | III.10 | III.14 |
| B16 | mouse melanoma | 36 | 2.1 | | |
| B16F | mouse melanoma, metastatic | 4.6 | 4.7 | | |
| SW620 | human colon cancer, metastasis | 250 | 1.2 | | |
| U87MG | human glioblastoma | 250 | 5.1 | | |
| HepG2 | human hepatocellular carcinoma | 3.6 | 1.7 | | |
| A549 | human lung adenocarcinoma | 236 | 1.0 | | |
| MCF-7 | human breast cancer, estrogen dependent, p53+/+, Rb +/+ | 194 | 2.3 | | |
| U20S | human osteosarcoma, p53+/-, Rb +/- | 250 | 1.5 | | |
| Saos2 | human rhabdomyosarcoma, p53-/-, Rb-/- | 250 | 1.8 | | |
| BT549 | human breast cancer, p53mut/mut | 250 | 2.0 | | |
| MDA-MB-238 | human breast cancer, estrogen independent, p53mut/mut | 195 | 1.4 | | |
| LNCaP | human prostate cancer, androgen dependent | 244 | 1.1 | | |
| DU145 | human prostate cancer, androgen independent, Rb-/- | 241 | 0.8 | | |

Table 2 (continued)

| Cytotoxic activity of selected betulinines against a panel of different (non)malignant cell lines. | | | | | |
|---|---|---|---|---|---|
| Cell Line | Description | Compound ($TCS_{50}$[mM]) | | | |
| | | Betulinic Acid | III.3 | III.10 | III.14 |
| HT-29 | human colon cancer | 250 | 1.6 | | |
| OVCAR-3 | human ovarian cancer | 164 | 1.0 | | |
| Caco-2 | human colon cancer | 20 | 3.0 | | |
| MEL-3 | human melanoma | 2.7 | 1.3 | | |
| Lymphocytes | human normal lymphocytes | 250 | 13 | | |
| NIH3T3 | mouse immortalised fibroblasts | 250 | 7.2 | | |
| K562 | human promyelocytic leukemia | 250 | 0.2 | | |
| K562-CdA | human promyelocytic leukemia, cladrubin resistant | 250 | 0.3 | | |
| K562-GEM | human promyelocytic leukemia, gemcitabin resistant | 101 1 | 0.9 | | |
| K562-ARA-C | human promyelocytic leukemia, cytarabin resistant | 250 | 0.6 | | |
| K562-FLUD | human promyelocytic leukemia, fludarabin resistant | 250 | 0.4 | | |
| CEM | human T-lymphoblastic leukemia | 250 | 1.0 | 13 | 170 |
| CEM-DNR 1/C2 | human T-lymphoblastic leukemia, daunorubicin resistant | 250 | 0.6 | | |
| CEM-DNR bulk | human T-lymphoblastic leukemia, daunorubicin resistant | 250 | 1.1 | | |
| CEM-VCR 1/F3 | human T-lymphoblastic leukemia, vincristin resistant | 19 | 3.3 | | |
| CEM-VCR 3/D5 | human T-lymphoblastic leukemia, vincristin resistant | 24 | 2.9 | | |
| CEM-VCR bulk | human T-lymphoblastic leukemia, vincristin resistant | 69 | 2.5 | | |

In contrast to betulinic acid, which is reported to be an agent selective for neuroectodermal derived tumours, there is no significant difference in the sensitivity of betulinines to tumours of different histogenetic origin.

The compounds are effective in submicromolar or low micromolar concentrations. However, the non-malignant cells, e. g. NIH3T3 fibroblasts and normal human lymphocytes, tolerated substantially higher doses of betulinines than the tumour cells suggesting a favourable therapeutic index.

Notably, the effectiveness of betulinines was found to be identical in cell lines bearing various mutations or deletions in cell cycle associated proteins (Table 2). This indicates that these substances should be equally effective in tumours with various alterations of tumour suppressor genes, namely p53, Rb, etc.

Furthermore, betulinines were shown to be equally effective in drug resistant cell lines as on their maternal counterparts, thereby suggesting that classical mechanisms of multidrug resistance apparently do not apply to these compounds. This particular characteristic should be of significant therapeutic benefit to chemotherapy resistant cancer patients.

Finally, the cytotoxic activity of betulinines is independent of the hormonal status of cancer cells, so the compounds should be equally effective in treatment of hormone dependent and independent cancers.

Those skilled in the art will recognise, or be able to ascertain using no more than routine experimentation, many equivalents to the specific embodiments of the invention described herein. Such equivalents are intended to be encompassed by the claims.

## Claims

**1.** A compound of formula I, or a pharmaceutically acceptable salt thereof, for use in therapy

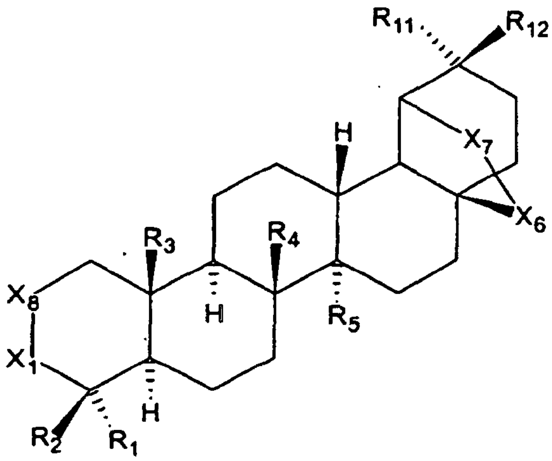

I

wherein:

$X^1$ is C=O;
$X^6$ is C=O, $CH_2$;
$X^7$ is O;
$X^8$ is C=O, C=NOR$^{1b}$, CHCN, CHOR$^{1b}$, CHOCOR$^{1b}$, CHOC(O)OR$^{1b}$, CHOC(O)OR$^{13}$, $CHOSO_2CH_3$, $CHOSO_2C_6H_4CH_3$, CH-Hal, C-(Hal)$_2$, $CHN_3$, $CH_2$, C=C(R$^{1b}$)$_2$, C=CR$^{1b}$OR$^{1b}$;
$R^{2-5}$ and $R^{11}$, $R^{12}$ are Me,
and wherein $R^1$ is H or $C_1$-$C_6$ alkyl, and Hal is Br, Cl, I, F;
$R^{1a-1b}$ are the same or different groups of $R^1$
$R^{13}$ is an OH-substituted alkyl group, an ether group or acyclic ether.

**2.** A compound according to claim 1 wherein:

$X^8$ is C=O, CH-Hal, CHOR$^{1a}$, CHOCOR$^{1a}$, C=C(R$^{1b}$)$_2$, C=CR$^{1b}$OR$^{1b}$, C-Hal$_2$, $CHN_3$ or CHCOOR$^{13}$.

**3.** A compound according to claim 1 or claim 2 wherein $R^{13}$ is a diol, triol or polyol.

**4.** A compound according to claim 1 or claim 2 wherein $R^{13}$ is glyceryl, 2,2-dimethyl-1,3-dioxolan-4-yl or pentaerythrityl.

**5.** A compound according to any preceding claim wherein:

$X^8$ is selected from C=O, CHBr, CHOH, CHOAc, C=$CH_2$, C=CHOH, C-$Br_2$, $CHN_3$, CHOC(O)$OCH_2CHOHCH_2OH$ or CHOC(O)$OCH_2$-2,2-dimethyl-1,3-dioxolan-4-yl.

**6.** A compound according to any preceding claim wherein the compound of formula I is selected from the following table:

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.3 | C=O | $CH_2$ | O | CHBr | Me |
| III.4 | C=O | $CH_2$ | O | CHOH | Me |
| III.5 | C=O | $CH_2$ | O | CHOAc | Me |
| III.10 | C=O | $CH_2$ | O | $CHN_3$ | Me |

(continued)

| No. | $X^1$ | $X^6$ | $X^7$ | $X^8$ | $R^{1-5}$, $R^{11,12}$ |
|---|---|---|---|---|---|
| III.13 | C=O | $CH_2$ | O | $CBr_2$ | Me |
| III.14 | C=O | $CH_2$ | O | $CF_2$ | Me |
| III.16 | C=O | $CH_2$ | O | CHF | Me |
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C=CH_2$ | Me |
| III.32 | C=O | $CH_2$ | O | C=CHOH | Me |

where

A is $CHOC(O)OCH_2CHOHCH_2OH$ and
B is $CHOC(O)OCH_2$-2,2-dimethyl-1,3-dioxolan-4-yl.

**7.** A compound according to any preceding claim wherein said compound is

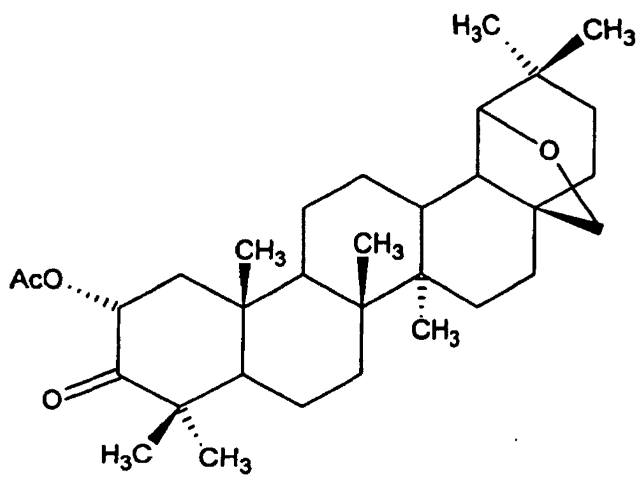

**8.** A compound according to any preceding claim for treating a proliferative disorder.

**9.** Use of a compound of formula I, or a pharmaceutically acceptable salt thereof, for manufacture of medicament for use in the treatment of a proliferative disorder.

**10.** A compound of formula Ia, or a pharmaceutically acceptable salt thereof,

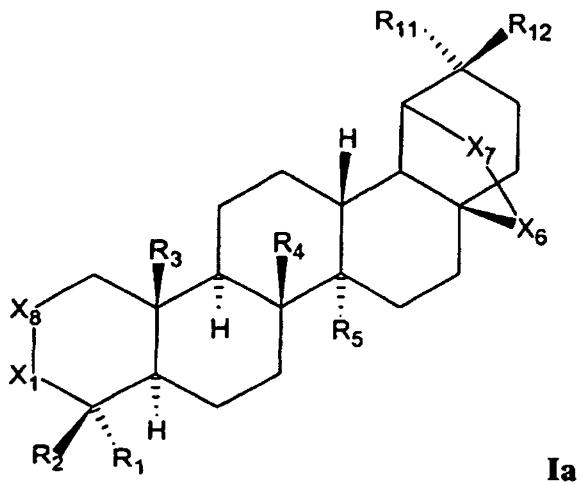

Ia

wherein:

$X^1$ is C=O;
$X^6$ is C=O, $CH_2$;
$X^7$ is O;
$X^8$ is C=O, C=NOR$^{1b}$, CHCN, CHOR$^{1b}$, CHOCOR$^{1b}$, CHOC(O)OR$^{1b}$, CHOC(O)OR$^{13}$, $CHOSO_2CH_3$, $CHOSO_2C_6H_4CH_3$, CH-Hal, C-(Hal)$_2$, $CHN_3$, C=C(R$^{1b}$)$_2$, C=CR$^{1b}$OR$^{1b}$;
$R^{2-5}$ and $R^{11}$, $R^{12}$ are Me,
and wherein R' is H or $C_1$-$C_6$ alkyl, and Hal is Br, Cl, I, F;
$R^{1a-1b}$ are the same or different groups of $R^1$;
$R^{13}$ is an OH-substituted alkyl group, an ether group or a cyclic ether.

with the proviso that when $X^6$ is $CH_2$, $X^7$ is O, $R^{1-5}$, $R^{11}$ and $R^{12}$ are Me;
$X^8$ is other than C=O, C=NOH, C=CHOH, CHBr, CHOH, CHOAc, CHCl, CHOMe or CHOEt.

**11.** A compound according to claim 10 wherein:

$X^8$ is C=O, CH-Hal, CHOR$^{1a}$, CHOCOR$^{1a}$, C=C(R$^{1b}$)$_2$, C=CR$^{1b}$OR$^{1b}$, C-Hal$_2$, $CHN_3$ or CHCOOR$^{13}$.

**12.** A compound according to claim 10 or 11 wherein $R^{13}$ is a diol, triol or polyol.

**13.** A compound according to claim 10 or claim 11 wherein $R^{13}$ is glyceryl, 2,2-dimethyl-1,3-dioxolan-4-yl or pentaerythrityl.

**14.** A compound according to any one of claims 10 to 13 wherein:

$X^8$ is selected from C=O, CH-Br, CHOH, CHOAc, C=$CH_2$, C=CHOH, C-$Br_2$, $CHN_3$, CHOC(O)$OCH_2CHOHCH_2$0H or CHOC(O)$OCH_2$-2,2-dimethyl-1,3-dioxolan-4-yl.

**15.** A compound according to any one of claims 10 to 14 selected from the following table:

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.10 | C=O | $CH_2$ | O | $CHN_3$ | Me |
| III.13 | C=O | $CH_2$ | O | $CBr_2$ | Me |
| III.14 | C=O | $CH_2$ | O | $CF_2$ | Me |
| III.16 | C=O | $CH_2$ | O | CHF | Me |

(continued)

| No. | $X^1$ | $X^6$ | $X^7$ | $X^8$ | $R^{1-5}$, $R^{11,12}$ |
|---|---|---|---|---|---|
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C=CH_2$ | Me |

where

A is $CHOC(O)OCH_2CHOHCH_2OH$ and
B is $CHOC(O)OCH_2$-2,2-dimethyl-1,3-dioxolan-4-yl.

**16.** A process for preparing a compound of formula I, as defined in claim 1, wherein $X^6$ is C=O, comprising oxidising a compound of formula Ib to a compound of formula Ic

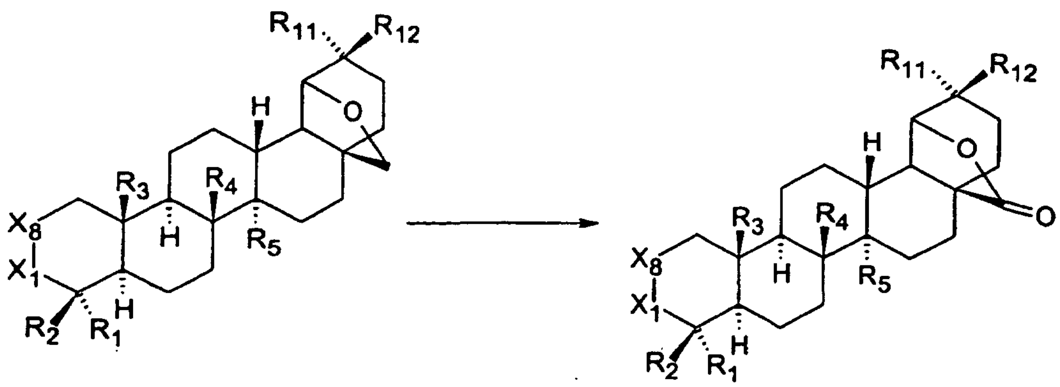

Ib Ic

**17.** A process according to claim 16 wherein the compound of formula Ib is oxidised to a compound of formula Ic by treating with chromium trioxide.

**18.** A process for preparing a compound of formula I, as defined in claim 1, wherein $R^1$-$R^5$, $R^{11}$, $R^{12}$ are methyl, $X^7$ is O, $X^6$ is $CH_2$, comprising reacting a compound of formula Id with sodium azide to form a compound of formula Ie

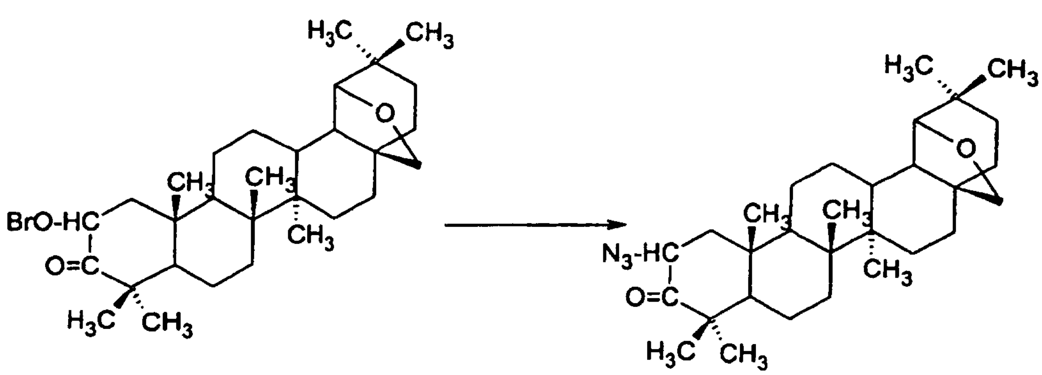

Id Ie

**19.** A process for preparing a compound of formula I, as defined in claim 1, wherein $R^1$-$R^5$, $R^{11}$, $R^{12}$ are methyl, $X^7$ is O, $X^6$ is $CH_2$, comprising

(i) reacting a compound of formula If with chloroformate of solketal to form a compound of formula Ig;
(ii) deprotecting said compound of formula Ig to form a compound of formula Ih

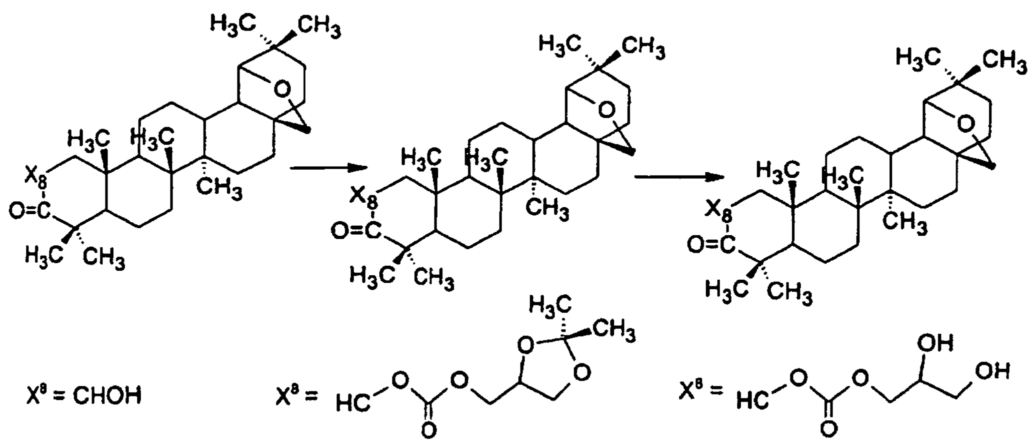

If Ig Ih

**20.** A process for preparing a compound of formula I, as defined in claim 1, wherein $R^1$-$R^5$, $R^{11}$, $R^{12}$ are methyl, $X^7$ is O, $X^6$ is $CH_2$, comprising reacting a compound of formula Ii with diethylaminosulphur trifluoride to form a compound of formula Ij

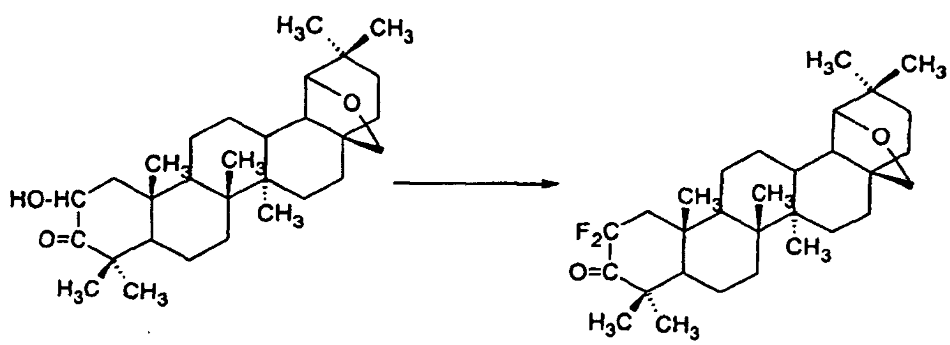

Ii Ij

## Patentansprüche

**1.** Verbindung der Formel I oder ein pharmazeutisch verträgliches Salz davon zur Verwendung in der Therapie

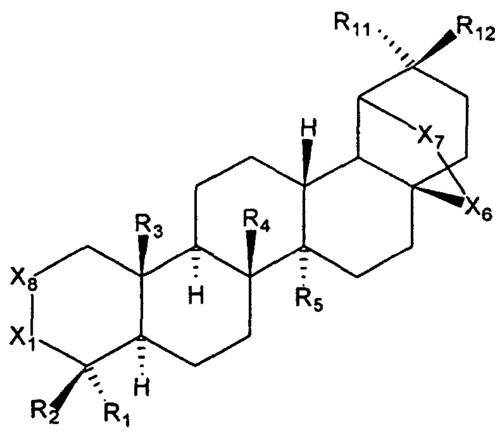

I

wobei

$X^1$ C=O ist;
$X^6$ C=O, $CH_2$ ist;
$X^7$ O ist;
$X^8$ C=O, C=NOR$^{1b}$, CHCN, CHOR$^{1b}$, CHOCOR$^{1b}$, CHOC(O)OR$^{1b}$, CHOC(O)OR$^{13}$, $CHOSO_2CH_3$, $CHOSO_2C_6H_4CH_3$, CH-Hal, C-(Hal)$_2$, $CHN_3$, $CH_2$, C=C(R$^{1b}$)$_2$, C=CR$^{1b}$OR$^{1b}$ ist;
$R^{2-5}$ und $R^{11}$, $R^{12}$ Me sind,
und wobei $R^1$ H oder $C_1$-$C_6$ -Alkyl ist und Hal Br, Cl, I, F ist;
$R^{1a-1b}$ gleiche oder verschiedene Gruppen von $R^1$ sind,
$R^{13}$ eine OH-substituierte Alkylgruppe, eine Ethergruppe oder ein cyclischer Ether ist.

2. Verbindungen nach Anspruch 1 wobei

$X^8$ C=O, CH-Hal, CHOR$^{1a}$, CHOCOR$^{1a}$, C=C(R$^{1b}$)$_2$, C=CR$^{1b}$OR$^{1b}$, C-Hal$_2$, $CHN_3$ oder CHCOOR$^{13}$ ist.

3. Verbindung nach Anspruch 1 oder Anspruch 2, wobei $R^{13}$ ein Diol, Triol oder Polyol ist.

4. Verbindung nach Anspruch 1 oder Anspruch 2, wobei $R^{13}$ Glyceryl, 2,2-Dimethyl-1,3-dioxolan-4-yl oder Pentaerythrityl ist.

5. Verbindung nach einem der vorhergehenden Ansprüche, wobei

$X^8$ unter C=O, CHBr, CHOH, CHOAc, C=$CH_2$, C=CHOH, C-$Br_2$, $CHN_3$, CHOC(O)$OCH_2CHOHCH_2OH$ oder CHOC(O)$OCH_2$-2,2-Dimethyl-1,3-dioxolan-4-yl ausgewählt ist.

6. Verbindung nach einem der vorhergehenden Ansprüche, wobei die Verbindung der Formel I aus der folgenden Tabelle ausgewählt ist:

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.3 | C=O | $CH_2$ | O | CHBr | Me |
| III.4 | C=O | $CH_2$ | O | CHOH | Me |
| III.5 | C=O | $CH_2$ | O | CHOAc | Me |
| III.10 | C=O | $CH_2$ | O | $CHN_3$ | Me |

(fortgesetzt)

| No. | $X^1$ | $X^6$ | $X^7$ | $X^8$ | $R^{1-5}$, $R^{11,12}$ |
|---|---|---|---|---|---|
| III.13 | C=O | $CH_2$ | O | $CBr_2$ | Me |
| III.14 | C=O | $CH_2$ | O | $CF_2$ | Me |
| III.16 | C=O | $CH_2$ | O | CHF | Me |
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C=CH_2$ | Me |
| III.32 | C=O | $CH_2$ | O | C=CHOH | Me |

in welcher

A $CHOC(O)OCH_2CHOHCH_2OH$ und
B $CHOC(O)OCH_2$-2,2-Dimethyl-1,3-dioxolan-4-yl ist.

**7.** Verbindung nach einem der vorhergehenden Ansprüche, wobei die genannte Verbindung eine Verbindung der Formel

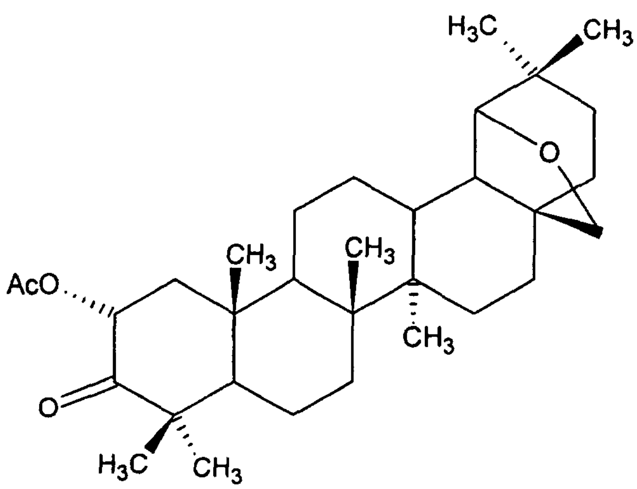

ist.

**8.** Verbindung nach einem der vorhergehenden Ansprüche zur Behandlung einer proliferativen Störung.

**9.** Verwendung einer Verbindung der Formel I oder eines pharmazeutisch verträglichen Salzes davon zur Herstellung eines Medikamentes zur Verwendung bei der Behandlung einer proliferativen Störung.

**10.** Verbindung der Formel Ia oder ein pharmazeutisch verträgliches Salz davon

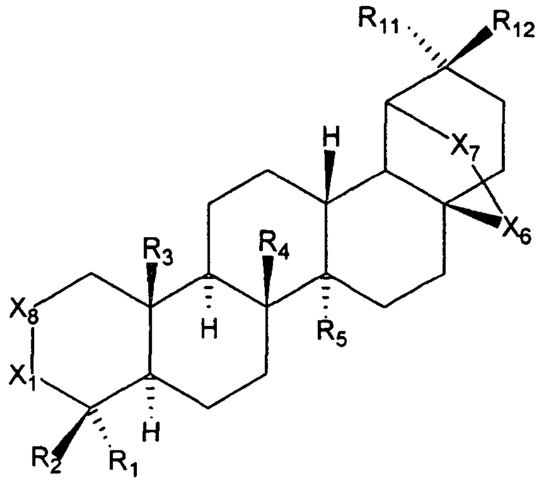

**Ia**

wobei:

$X^1$ C=O ist;
$X^6$ C=O, $CH_2$ ist;
$X^7$ O ist;
$X^8$ C=O, C=NOR$^{1b}$, CHCN, CHOR$^{1b}$, CHOCOR$^{1b}$, CHOC(O)OR$^{1b}$, CHOC(O)OR$^{13}$, $CHOSO_2CH_3$, $CHOSO_2C_6H_4CH_3$, CH-Hal, C-(Hal)$_2$, $CHN_3$, C=C(R$^{1b}$)$_2$, C=CR$^{1b}$OR$^{1b}$ ist;
$R^{2-5}$ und $R^{11}$, $R^{12}$ Me sind,
und wobei $R^1$ H oder $C_1$-$C_6$ - Alkyl ist, und Hal Br, Cl, I, F ist;
$R^{1a-1b}$ gleiche oder verschiedene Gruppen von $R^1$ sind;
$R^{13}$ eine OH-substituierte Alkylgruppe, eine Ethergruppe oder ein cyclischer Ether ist;

mit der Maßgabe, dass wenn $X^6$ $CH_2$ ist, $X^7$ O ist, $R^{1-5}$, $R^{11}$ und $R^{12}$ Me sind; $X^8$ nicht C=O, C=NOH, C=CHOH, CHBr, CHOH, CHOAc, CHCl, CHOMe oder CHOEt ist.

**11.** Verbindung nach Anspruch 10, wobei

$X^8$ C=O, CH-Hal, CHOR$^{1a}$, CHOCOR$^{1a}$, C=C(R$^{1b}$)$_2$, C=CR$^{1b}$OR$^{1b}$, C-Hal$_2$, $CHN_3$ oder CHCOOR$^{13}$ ist.

**12.** Verbindung nach Anspruch 10 oder 11, wobei $R^{13}$ ein Diol, Triol oder Polyol ist.

**13.** Verbindung nach Anspruch 10 oder Anspruch 11, wobei $R^{13}$ Glyceryl, 2,2-Dimethyl-1,3-dioxolan-4-yl oder Pentaerythrityl ist.

**14.** Verbindung nach einem der Ansprüche 10 bis 13, wobei

$X^8$ unter C=O, CH-Br, CHOH, CHOAc, C=$CH_2$, C=CHOH, C-$Br_2$, $CHN_3$, CHOC(O)O$CH_2$CHOH$CH_2$OH oder CHOC(O)O$CH_2$-2,2-Dimethyl-1,3-dioxolan-4-yl. ausgewählt ist.

**15.** Verbindung nach einem der Ansprüche 10 bis 14, die aus der folgenden Tabelle ausgewählt ist:

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.10 | C=O | $CH_2$ | O | $CHN_3$ | Me |
| III.13 | C=O | $CH_2$ | O | $CBr_2$ | Me |
| III.14 | C=O | $CH_2$ | O | $CF_2$ | Me |

(fortgesetzt)

| No. | $X^1$ | $X^6$ | $X^7$ | $X^8$ | $R^{1-5}$, $R^{11,12}$ |
|---|---|---|---|---|---|
| III.16 | C=O | $CH_2$ | O | CHF | Me |
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C=CH_2$ | Me |

in welcher

A $CHOC(O)OCH_2CHOHCH_2OH$ und
B $CHOC(O)OCH_2$-2,2-Dimethyl-1,3-dioxotan-4-yl ist.

**16.** Verfahren zur Herstellung einer Verbindung der Formel I, wie in Anspruch 1 definiert, wobei $X^6$ C=O ist, umfassend die Oxidation einer Verbindung der Formel Ib zu einer Verbindung der Formel Ic

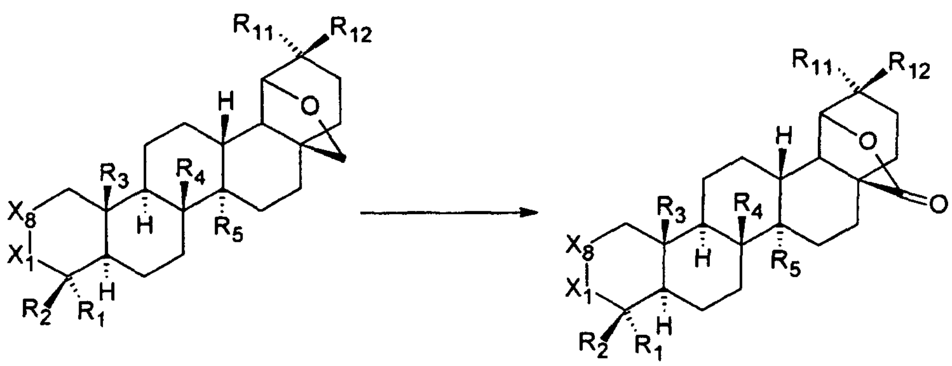

Ib Ic

**17.** Verfahren nach Anspruch 16, wobei die Verbindung der Formel Ib durch Behandeln mit Chromtrioxid zu einer Verbindung der Formel Ic oxidiert wird.

**18.** Verfahren zur Herstellung einer Verbindung der Formel I nach Anspruch 1, wobei $R^1$-$R^5$, $R^{11}$, $R^{12}$ Methyl sind, $X^7$ O ist, $X^6$ $CH_2$ ist, umfassend die Umsetzung einer Verbindung der Formel Id mit Natriumazid unter Bildung einer Verbindung der Formel Ie

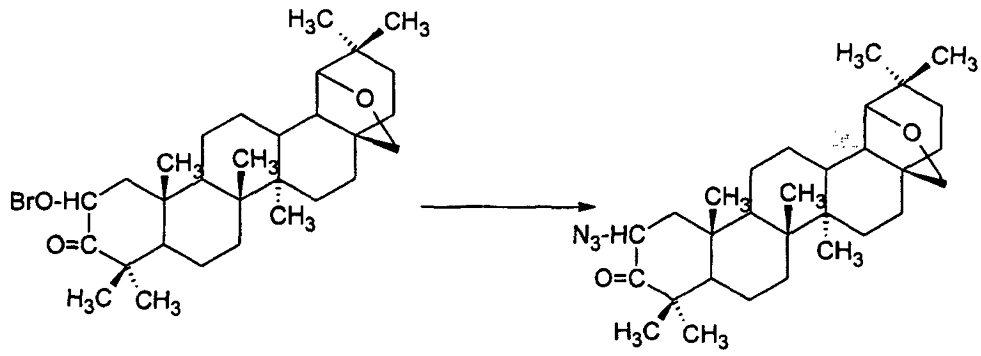

Id Ie

**19.** Verfahren zur Herstellung einer Verbindung der Formel I, wie in Anspruch 1 definiert, wobei $R^1$-$R^5$, $R^{11}$, $R^{12}$ Methyl sind, $X^7$ O ist, $X^6$ $CH_2$ ist, umfassend

(i) die Umsetzung einer Verbindung der Formel If mit Solketal-Chlorformiat unter Bildung einer Verbindung der Formel Ig und die

(ii) Entfernung der Schutzgruppe der Verbindung der Formel Ig unter Bildung einer Verbindung der Formel Ih

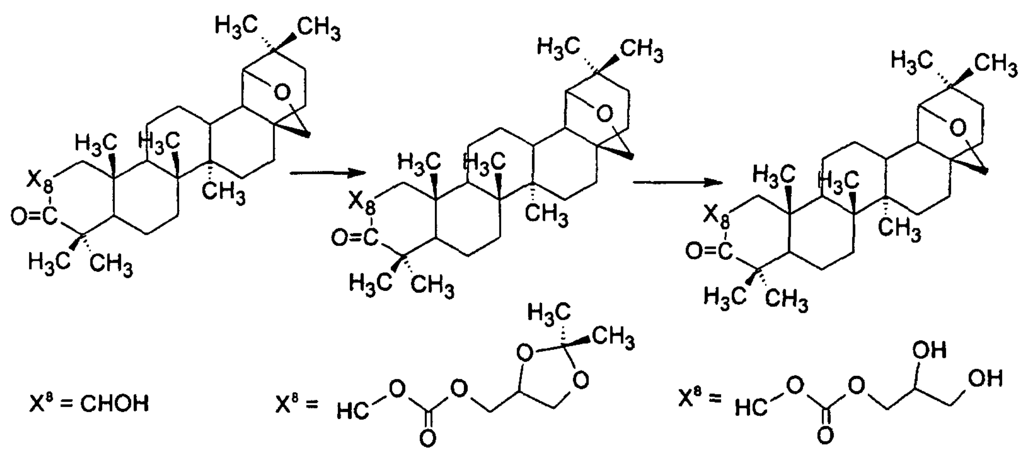

$X^8$ = CHOH

If Ig Ih

**20.** Verfahren zur Herstellung einer Verbindung der Formel I, wie in Anspruch 1 definiert, wobei $R^1$-$R^5$, $R^{11}$, $R^{12}$ Methyl sind, $X^7$ O ist, $X^6$ $CH_2$ ist, umfassend die Umsetzung einer Verbindung der Formel Ii mit Diethylaminoschwefeltrifluorid unter Bildung einer Verbindung der Formel Ij.

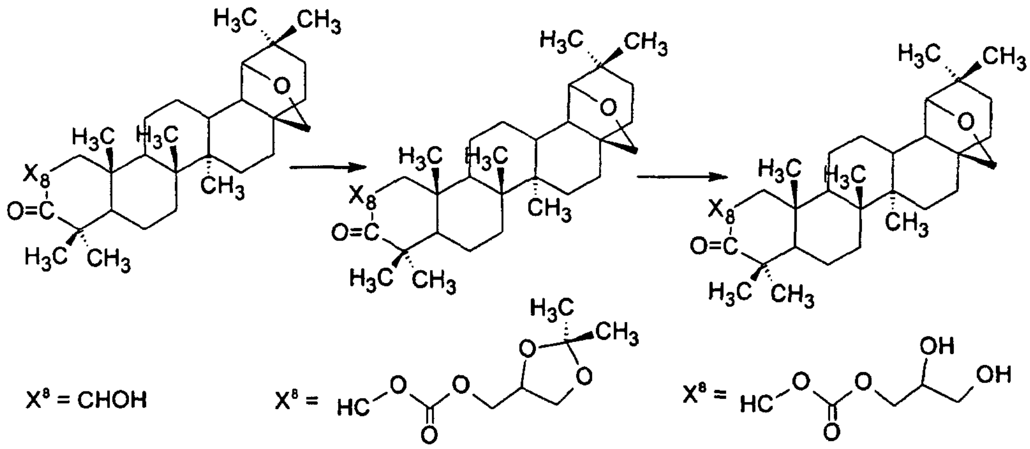

$X^8$ = CHOH

$X^8$ =

$X^8$ =

If Ig Ih

**Revendications**

1. Composé de formule I ou un de ses sels pharmaceutiquement acceptables, destiné à être utilisé en thérapeutique,

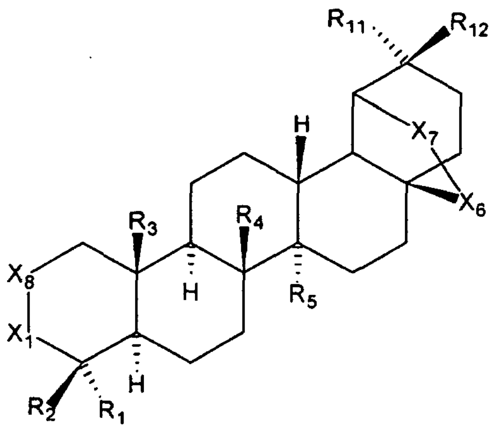

I

formule dans laquelle:

$X^1$ représente un groupe C=O;
$X^6$ represente un groupe C=O, $CH_2$;
$X^7$ représente un atome de O;
$X^8$ représente un groupe C=O, C=NOR$^{1b}$, CHCN, CHOR$^{1b}$, CHOCOR$^{1b}$, CHOC(O)OR$^{1b}$, CHOC(O)OR$^{13}$, $CHOSO_2CH_3$, $CHOSO_2C_6H_4CH_3$, CH-Hal, C-(Hal)$_2$, $CHN_3$, $CH_2$, C=C(R$^{1b}$)$_2$, C=CR$^{1b}$OR$^{1b}$;
$R^{2-5}$ et $R^{11}$, $R^{12}$ représentent des groupes Me,
et dans laquelle $R^1$ représente H ou un groupe alkyle en $C_1$ à $C_6$ et Hal représente Br, Cl, I ou F;
$R^{1a-1b}$ sont des groupes identiques ou différents choisis parmi les groupes de $R^1$;
$R^{13}$ représente un groupe alkyle à substituant -OH, un groupe éther ou un éther cyclique.

2. Composé selon la revendication 1, dans lequel :

$X^8$ représente un groupe C=O, CH-Hal, CHOR$^{1a}$, CHOCOR$^{1a}$, C=C(R$^{1b}$)$_2$, C=CR$^{1b}$OR$^{1b}$, C-Hal$_2$, $CHN_3$ ou

CHCOOR$^{13}$.

**3.** Composé selon la revendication 1 ou la revendication 2, dans lequel $R^{13}$ représente un groupe diol, triol ou polyol.

**4.** Composé selon la revendication 1 ou la revendication 2, dans lequel $R^{13}$ représente un groupe glycéryle, 2,2-diméthyl-1,3-dioxolanne-4-yle ou pentaérythrityle.

**5.** Composé selon l'une quelconque des revendications précédentes, dans lequel :

$X^8$ est choisi entre des groupes C=O, CHBr, CHOH, CHOAc, $C{=}CH_2$, C=CHOH, C-$Br_2$, $CHN_3$, CHOC(O)$OCH_2CHOHCH_2OH$ et CHOC(O)$OCH_2$-2,2-diméthyl-1,3-dioxolanne-4-yle.

**6.** Composé selon l'une quelconque des revendications précédentes, les composés de formule I étant chisis parmi ceux du tableau suivant:

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.3 | C=O | $CH_2$ | O | CHBr | Me |
| III.4 | C=O | $CH_2$ | O | CHOH | Me |
| III.5 | C=O | $CH_2$ | O | CHOAc | Me |
| III.10 | C=O | $CH_2$ | O | $CHN_3$ | Me |
| III.13 | C=O | $CH_2$ | O | $CBr_2$ | Me |
| III.14 | C=O | $CH_2$ | O | $CF_2$ | Me |
| III.16 | C=O | $CH_2$ | O | CHF | Me |
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C{=}CH_2$ | Me |
| III.32 | C=O | $CH_2$ | O | C=CHOH | Me |

où

A représente un groupe CHOC(O)$OCH_2CHOHCH_2OH$ et
B représente un groupe CHOC(O)$OCH_2$-2,2-diméthyl-1,3-dioxolanne-4-yle.

**7.** Composé selon l'une quelconque des revendications précédentes, ledit composé répondant à la formule

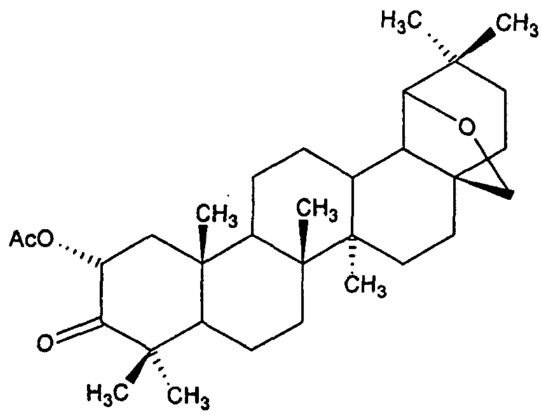

8. Composé selon l'une quelconque des revendications précédentes pour le traitement d'un trouble prolifératif.

9. Utilisation d'un composé de formule I ou d'un de ses sels pharmaceutiquement acceptables, pour la production d'un médicament destiné à être utilisé dans le traitement d'un trouble prolifératif.

10. Composé de formule Ia, ou d'un de ses sels pharmaceutiquement acceptables,

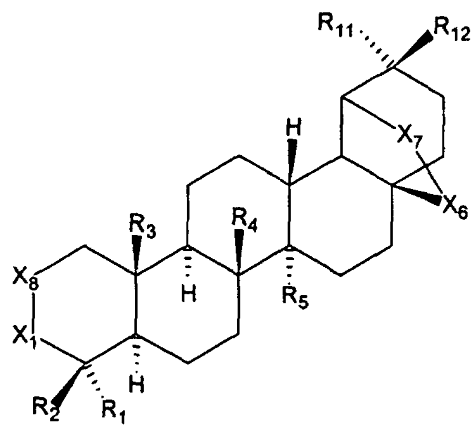

**Ia**

formule dans laquelle:

$X^1$ représente un groupe C=O;
$X^6$ représente un groupe C=O, $CH_2$;
$X^7$ représente un atome de O;
$X^8$ représente un groupe C=O, C=NOR$^{1b}$, CHCN, CHOR$^{1b}$, CHOCOR$^{1b}$, CHOC(O)OR$^{1b}$, CHOC(O)OR$^{13}$, $CHOSO_2CH_3$, $CHOSO_2C_6H_4CH_3$, CH-Hal, C-$(Hal)_2$, $CHN_3$, C=C$(R^{1b})_2$, C=CR$^{1b}$OR$^{1b}$;
$R^{2-5}$ et $R^{11}$, $R^{12}$ représentent des groupes Me,
et dans laquelle $R^1$ représente H ou un groupe alkyle $C_1$ à $C_6$ et Hal représente Br, Cl, I ou F;
$R^{1a-1b}$ sont des groupes identiques ou différents chisis parmi les groupes de $R^1$;
$R^{13}$ représent un groupe alkyle à substituant -OH, un groupe éther ou un éther cyclique;

sous réserve que, lorsque $X^6$ représente un groupe $CH_2$, $X^7$ représente un atome de O, $R^{1-5}$, $R^{11}$ et $R^{12}$ représentent des groupes Me;

$X^8$ est autre qu'un groupe C=O, C=NOH, C=CHOH, CHBr, CHOH, CHOAc, CHCl, CHOMe ou CHOEt.

**11.** Composé selon la revendication 10, dans lequel

$X^8$ représente un groupe C=O, CH-Hal, $CHOR^{1a}$, $CHOCOR^{1a}$, $C=C(R^{1b})_2$, $C=CR^{1b}OR^{1b}$, $C\text{-}Hal_2$, $CHN_3$ ou $CHCOOR^{13}$.

**12.** Composé selon la revendication 10 ou la revendication 11, dans lequel $R^{13}$ représente un groupe diol, triol ou polyol.

**13.** Composé selon la revendication 10 ou la revendication 11, dans lequel $R^{13}$ représente glycéryle, 2,2-diméthyl-1,3-dioxolanne-4-yle ou pentaérythrityle.

**14.** Composé selon l'une quelconque des revendications 10 à 13, dans lequel :

$X^8$ est choisi entre des groupes C=O, CH-Br, CHOH, CHOAc, $C=CH_2$, C=CHOH, $C\text{-}Br_2$, $CHN_3$, CHOC(O)$OCH_2CHOHCH_2OH$ ou CHOC(O)$OCH_2$-2,2-diméthyl-1,3-dioxolanne-4-yle.

**15.** Composé selon l'une quelconque des revendications 10 à 14, choisi parmi les composés du tableau suivant:

| No. | $X^1$ | $X^6$ | $X^7$ | $X^8$ | $R^{1-5}$, $R^{11,12}$ |
|---|---|---|---|---|---|
| III.2 | C=O | C=O | O | CHOAc | Me |
| III.10 | C=O | $CH_2$ | O | $CHN_3$ | Me |
| III.13 | C=O | $CH_2$ | O | $CBr_2$ | Me |
| III.14 | C=O | $CH_2$ | O | $CF_2$ | Me |
| III.16 | C=O | $CH_2$ | O | CHF | Me |
| III.21 | C=O | $CH_2$ | O | A | Me |
| III.22 | C=O | $CH_2$ | O | B | Me |
| III.29 | C=O | C=O | O | CHBr | Me |
| III.30 | C=O | C=O | O | $CBr_2$ | Me |
| III.31 | C=O | $CH_2$ | O | $C=CH_2$ | Me |

où

A représente un groupe CHOC(O)$OCH_2CHOHCH_2OH$ et
B représente un groupe CHOC(O)$OCH_2$-2,2-diméthyl-1,3-dioxolanne-4-yle.

**16.** Procédé pour la préparation d'un composé de formule I, répondant à la définition dans la revendication 1, dans lequel $X^6$ représente un groupe C=O, comprenant l'oxydation d'un composé de formule Ib en un composé de formule Ic.

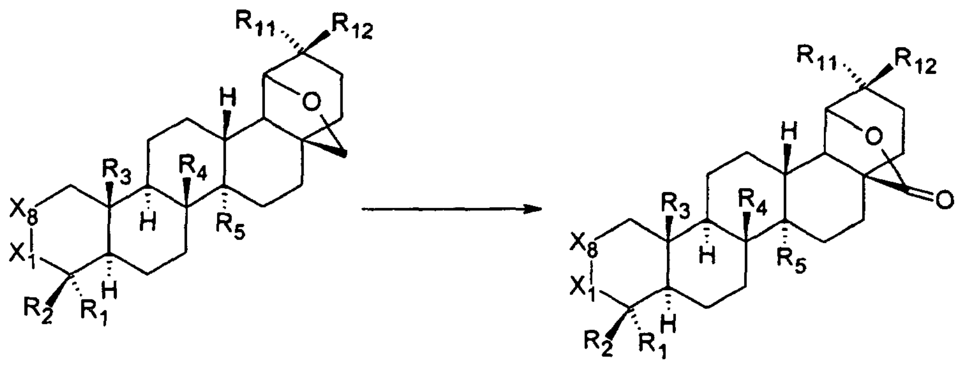

Ib Ic

17. Procédé selon la revendication 16, dans lequel le composé de formule Ib est oxydé en un composé de formule Ic par traitement avec du trioxyde de chrome.

18. Procédé pour la préparation d'un composé de formule I, répondant à la définition dans la revendication 1, dans lequel $R^1$-$R^5$, $R^{11}$, $R^{12}$ représentent des groupes méthyle, $X^7$ représente un atome de O, $X^6$ représente un groupe $CH_2$, comprenant la réaction d'un composé de formule Id avec de l'azoture de sodium pour former un composé de formule Ie.

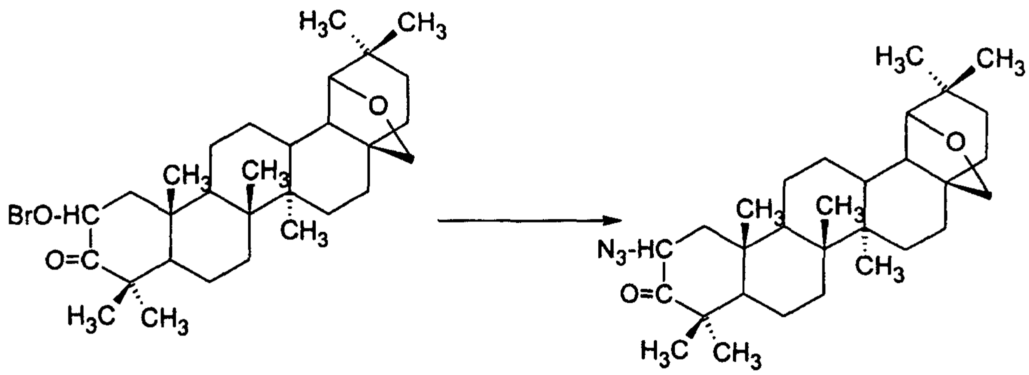

Id Ie

19. Procédé pour la préparation d'un composé de formule I, répondant à la définition dans la revendication 1, dans lequel $R^1$-$R^5$, $R^{11}$, $R^{12}$ représentent des groupes méthyle, $X^7$ représente un atome de O, $X^6$ représente un groupe $CH_2$, comprenant

(i) la réaction d'un composé de formule If avec du chloroformiate de solcétal pour former un composé de formule Ig;
(ii) la suppression de la protection dudit composé de formule Ig pour former un composé de formule Ih.

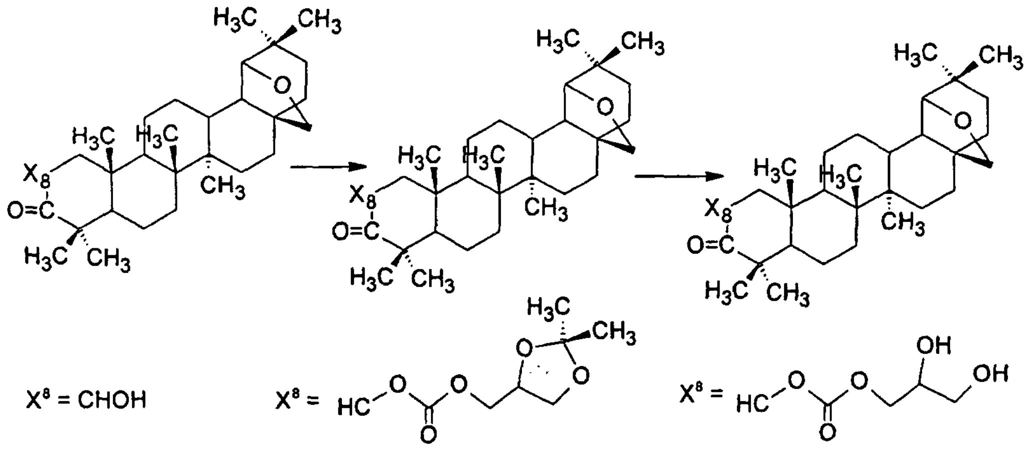

$X^8$ = CHOH

$X^8$ =

$X^8$ =

If Ig Ih

**20.** Procédé pour la préparation d'un composé de formule I, répondant à la définition dans la revendication 1, dans lequel $R^1$-$R^5$, $R^{11}$, $R^{12}$ représentent des groupes méthyle, $X^7$ représente un atome de O, $X^6$ représente un groupe $CH_2$, comprenant la réaction d'un composé de formule Ii avec du diéthylaminosulfotrifluorure pour former un composé de formule Ij.

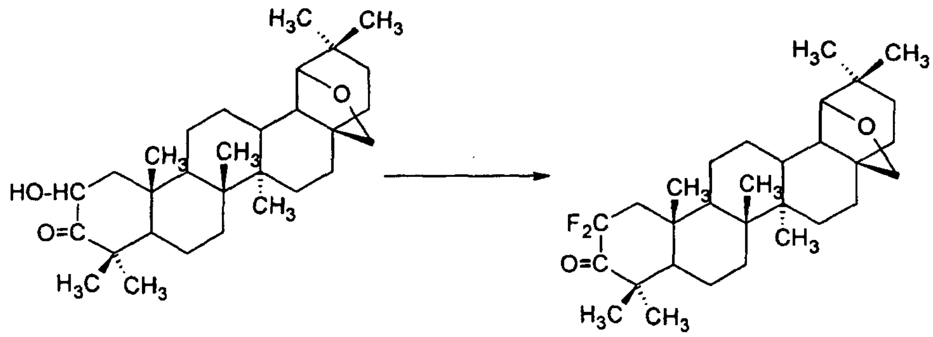

Ii Ij